# FASCICULE DE BREVET EUROPEEN

(11) **EP 3 467 856 B1**
(45) Date de publication et mention de la délivrance du brevet: **04.12.2019**
(21) Numéro de dépôt: 18196749.8
(22) Date de dépôt: 26.09.2018
(51) Int. Cl.: H01G 4/35, H01G 2/10, H02G 3/22, H01G 4/236, A61N 1/375

(54) **PROCÉDÉS DE FABRICATION D'UNE TRAVERSÉE HERMÉTIQUE ET ISOLANTE POUR UN BOITIER, NOTAMMENT EN TITANE, D'UN DISPOSITIF ÉLECTRONIQUE**
HERSTELLUNGSVERFAHREN EINER HERMETISCHEN UND ISOLIERENDEN DURCHFÜHRUNG FÜR EIN GEHÄUSE, INSBESONDERE AUS TITAN, EINER ELEKTRONISCHEN VORRICHTUNG
PROCESS OF MANUFACTURING A HERMETIC AND INSULATING FEEDTHROUGH FOR A HOUSING, ESPECIALLY TITANIUM, OF AN ELECTRONIC DEVICE

(30) Priorité: 03.10.2017 FR 1759208
(43) Date de publication de la demande: 10.04.2019
(73) Titulaire: Mistic, 92130 Issy Les Moulineaux (FR)
(72) Inventeur: BOUTAUD, Bertrand, 92130 Issy les Moulineaux (FR)
(74) Mandataire: Dupuis-Latour, Dominique

(56) Documents cités:
- EP-A1- 3 476 284
- US-A1- 2011 139 484
- US-A1- 2015 165 218

## Description

L'invention concerne la technique de conception et de fabrication de composants ou parties de composants dénommés "traversées", qui sont des passages hermétiques et électriquement isolés d'une liaison électrique au travers d'une paroi métallique.

La paroi métallique peut être notamment celle du boitier d'un dispositif électronique, les traversées permettant alors d'assurer des liaisons électriques entre le volume intérieur du boitier (côté interne de la traversée), qui par exemple loge des circuits électroniques, et une face apparente de ce même boitier (côté externe de la traversée).

La liaison électrique comprend un élément traversant métallique, conducteur. Le boitier étant métallique, donc également conducteur, il est nécessaire de prévoir au niveau de la traversée une interface d'isolation électrique entre élément traversant et boitier, cette interface procurant en outre une herméticité parfaite pour préserver l'étanchéité du volume intérieur du boitier.

Un exemple - non limitatif - de telles traversées peut être trouvé dans les dispositifs médicaux actifs, notamment ceux destinés à être implantés dans l'organisme de façon permanente.

Ces dispositifs sont souvent conçus sous la forme d'un générateur constitué d'un boitier métallique, généralement en titane, sur lequel est montée une tête de connecteur pourvue de logements permettant de raccorder mécaniquement et électriquement au boitier du générateur une ou plusieurs sondes reliées à des électrodes distantes.

La liaison des contacts de la tête de connecteur aux divers circuits électroniques enfermés dans le boitier implique la réalisation de plusieurs traversées de ce boitier, avec pour chacune un picot destiné à être relié d'une part à une borne correspondante de la tête de connecteur par son extrémité émergente (côté externe), et d'autre part aux circuits électroniques par son extrémité opposée (côté interne) débouchant dans le volume intérieur du boitier où se trouvent ces circuits.

De telles traversées sont par exemple décrites dans les EP 2 377 573 A1 et EP 2 873 437 A1 (Sorin CRM).

Ces traversées peuvent être également rencontrées dans des sous-composants de dispositifs électroniques, médicaux ou autres, tels que les batteries et les condensateurs. Les documents US2011/139484A1 et US2014/020951A1 décrivent une technique permettant de réaliser un découplage physique et électrique entre l'élément traversant et le substrat métallique qui l'entoure et dans lequel il est intégré, sans pont d'ancrage par une couche isolante rapportée en surface. Cette technique consiste à réaliser sous forme monolithique un îlot central conducteur isolé du reste du substrat sur toute son épaisseur par une couche latérale périphérique de matériau isolant remplissant l'intégralité d'une rainure circonférentielle préalablement creusée pour définir l'îlot central détouré dans le substrat.

Dans le cas particulier d'un dispositif médical implanté, la traversée doit non seulement isoler électriquement le conducteur traversant (picot ou autre) du boitier métallique, mais doit être également parfaitement hermétique pour éviter toute pénétration de fluide à l'intérieur du boitier, et ceci pendant toute la durée de vie du dispositif implanté, typiquement pendant une dizaine d'années. La traversée est donc un des éléments clés des dispositifs médicaux implantables, car elle assure une double fonction de passage du courant électrique et d'étanchéité du boitier.

Les US 7 310 216 B2 (Greatbatch-Sierra) et US 2017/0203105 A1 (Biotronik) décrivent de telles traversées conçues pour des dispositifs médicaux implantables actifs et incorporant éventuellement, outre les liaisons électriques, des structures de filtrage ou de découplage de ces liaisons électriques.

Les technologies mises en œuvre sont toutefois relativement complexes et impliquent une multiplicité d'étapes successives, non seulement pour la réalisation de la traversée mais aussi pour le montage ultérieur de chacune d'entre elles sur le boitier en garantissant une liaison mécanique la plus résistante et la plus fiable possible.

Outre la complexité - et par voie de conséquence le coût élevé - de ces technologies, celles-ci présentent encore un certain nombre d'inconvénients tenant notamment au fait qu'il n'est pas possible de rapprocher les unes des autres les différentes traversées adjacentes autant qu'il serait souhaitable.

Or la tendance à la miniaturisation et à l'augmentation du nombre de liaisons électriques, que ce soit dans le domaine des dispositifs médicaux ou dans de nombreux autres domaines, impose de réduire fortement le pas d'entraxe ou *pitch* entre traversées adjacentes.

Les EP 2 377 573 A1 et EP 2 873 437 A1 précités décrivent une manière de réaliser une traversée par mise en œuvre de technologies comparables à celles utilisées pour la fabrication des circuits intégrés monolithiques, avec gravure d'un substrat métallique et dépôt de couches sur le substrat ainsi gravé.

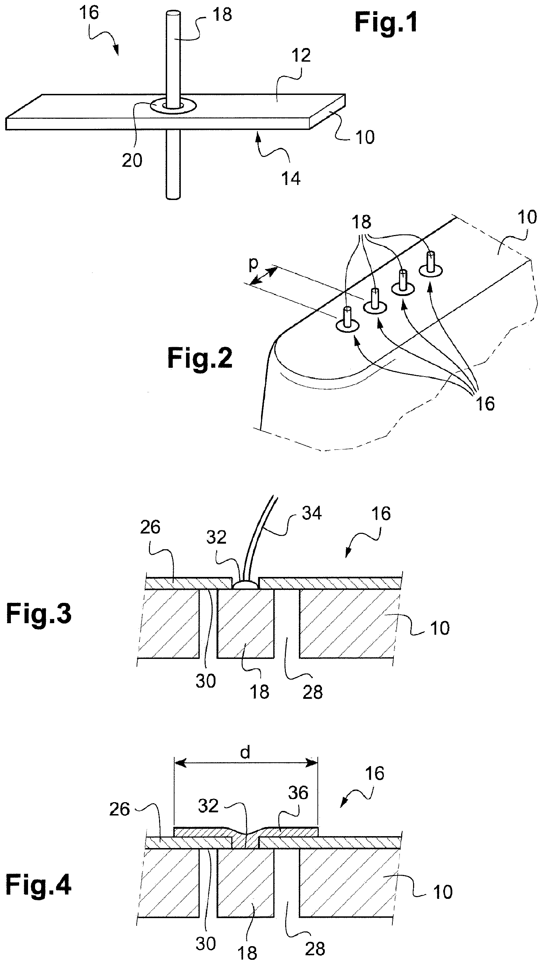

Ce type de traversée intégrée, qui sera décrite plus en détail par la suite en référence aux Figures 3 et 4, comprend un élément traversant détouré dans le substrat par gravure de ce dernier, et maintenu mécaniquement grâce à une couche isolante préalablement déposée en surface du substrat. Cette couche supérieure isolante de maintien couvre à la fois le substrat autour de l'élément traversant et l'élément traversant proprement dit, qui est alors suspendu par la couche supérieure isolante - ce qui assure le maintien mécanique - en une position permettant de laisser subsister sur la totalité de son contour périphérique un intervalle évidé - ce qui assure l'isolation électrique avec le reste du substrat.

Toutefois, pour assurer la solidité mécanique requise, la suspension par une ou plusieurs couches supérieures nécessite le dépôt de couches relativement épaisses (environ 10 µm par couche) par rapport aux procédés conventionnels de dépôt utilisés dans la technologie des circuits intégrés, ce qui s'avère coûteux.

Les couches épaisses peuvent également engendrer un niveau de contraintes mécaniques important et une déformation significative du substrat, ce qui peut rendre la fabrication délicate et influencer négativement la fiabilité de la structure obtenue.

Certes, pour renforcer la structure il est possible d'ajouter ultérieurement un matériau de remplissage de la cavité évidée, tel que colle ou résine. Ce palliatif implique toutefois une étape supplémentaire et accroit donc le coût de fabrication, et de plus conduit à manipuler des structures suspendues, encore assez fragiles malgré la pluralité de couches de support. Par ailleurs, si l'une des couches additionnelles de support est conductrice il est nécessaire d'intercaler entre deux traversées adjacentes un muret d'isolation, ce qui pénalise la réduction du pas d'entraxe entre les traversées.

Enfin, une interaction potentielle entre des couches additionnelles isolantes et conductrices peut conduire à une perte d'isolation électrique au fil du temps. Pour garantir les performances de la traversée pendant toute la durée de vie du dispositif, il peut être alors nécessaire d'ajouter des sous-couches intermédiaires qui limiteront les diffusions entre couches voisines, ce qui augmente encore plus la complexité de la structure et donc son coût de réalisation.

Les US 2011/139484 A1 et US 2014/020951 A1 décrivent une autre technique permettant de réaliser un découplage physique et électrique entre l'élément traversant et le substrat métallique qui l'entoure et dans lequel il est intégré, sans pont d'ancrage par une couche isolante rapportée en surface.

Cette technique consiste à réaliser sous forme monolithique un ilot central conducteur isolé du reste du substrat sur toute son épaisseur par une couche latérale périphérique de matériau isolant remplissant l'intégralité d'une rainure circonférentielle préalablement creusée pour définir l'ilot central détouré dans le substrat.

La mise en œuvre de cette autre technique souffre néanmoins de difficultés technologiques et conceptuelles antagonistes et irréconciliables.

En effet, un composant tel qu'une traversée intégrée à un boitier de dispositif implantable requiert une épaisseur d'au minimum 100 à 150 µm pour pouvoir exécuter les opérations d'assemblage entre les deux pièces et garantir à l'ensemble obtenu une étanchéité et une stabilité mécanique parfaites. Plus précisément, les procédés d'assemblage reposent le plus souvent sur une soudure laser (ou électrique) qui doit s'établir typiquement sur une profondeur minimale de 50 à 100 µm, avec une zone thermiquement affectée qui s'étend elle-même en général sur 50 µm au-delà de la soudure.

Pour répondre à ces exigences, la technique décrite par les deux documents précités impose d'une part de graver une rainure sur une profondeur au moins égale à l'épaisseur finale du composant, d'autre part de remplir cette rainure par un matériau isolant électrique répondant aux exigences d'étanchéité très strictes des dispositifs médicaux implantables. Or, sur ce dernier point il est bien connu que tous les procédés de dépôt, de couches épaisses supérieures à quelques micromètres engendrent des porosités et des stratifications incompatibles avec des exigences très strictes d'étanchéité, et ceci quels que soient les post-traitements effectués, dont ceux mentionnés dans les deux documents précités.

Enfin, pour assurer le maintien mécanique de l'ilot central pendant la réalisation de la traversée, les procédés décrits par ces deux documents prévoient toujours de graver une rainure initialement borgne, qui laisse donc subsister sur la fraction non gravée une continuité électrique entre l'ilot central et le reste du substrat. La suppression de cette continuité électrique doit être réalisée par un amincissement final du substrat sur une profondeur supérieure ou égale à la fraction non gravée.

Ce mécanisme d'amincissement final, qui a lieu sur toute la superficie du substrat, est de fait conceptuellement intrinsèque aux techniques décrites par les US 2011/139484 A1 et US 2014/020951 A1.

De plus, pour pouvoir être mises en œuvre, ces techniques nécessitent la gravure de microrainures sur une ouverture typiquement de quelques micromètres pour pouvoir les combler par des couches minces suffisamment denses - et cela sur une profondeur excédant 100 µm.

Or les technologies de gravure ne permettent pas d'obtenir des profils avec des facteurs de forme (ratio entre ouverture et profondeur de gravure) aussi faibles.

Le problème de l'invention est de remédier à ces inconvénients et limitations de l'état de la technique, par un nouveau moyen de réaliser une traversée monolithique à isolation verticale latérale micrométrique :
- qui puisse garantir un maintien mécanique robuste entre deux parties d'un même substrat, par des mécanismes originaux de maintien mécanique de l'ilot pendant et après le processus ;
- avec une séquence particulière d'étapes de fabrication qui, en particulier, ne nécessite pas d'amincissement général du substrat après les étapes de détourage de l'ilot et d'oxydation contrôlée, comme dans le cas des techniques connues exposées ci-dessus ;
- sur une épaisseur pouvant atteindre au moins 100 à 150 µm, compatible avec la réalisation de traversées intégrées à un boitier de dispositif implantable ;
- et ceci en garantissant à l'ensemble obtenu une étanchéité et une stabilité mécanique parfaites.

L'invention a également pour objet de permettre la réalisation de traversées multiples très rapprochées, présentant un pas d'entraxe extrêmement réduit entre traversées adjacentes, avec par voie de conséquence la possibilité de miniaturiser très fortement le dispositif dans lequel ces traversées sont intégrées et/ou de regrouper les traversées sur une zone d'étendue réduite, avec une densité de contacts très élevée.

L'invention permet également, comme on le verra, l'adjonction aux traversées de composants additionnels intégrés tels que condensateurs de découplage ou de filtrage, antenne RF, etc. réalisés en même temps que ces traversées.

À cet effet, l'invention propose un procédé de réalisation d'une traversée telle que celle décrite par le US 2011/139484 A1 précité, c'est-à-dire une traversée monolithique hermétique à isolation verticale latérale pour le passage d'une liaison électrique au travers d'une paroi métallique d'un dispositif électrique.

De façon caractéristique, le procédé de l'invention comprend les étapes suivantes :
a) obtention d'un substrat métallique dont l'épaisseur correspond à la dimension en direction transversale de la paroi comprenant la traversée à réaliser ;
b) amincissement du substrat, au moins dans une zone d'isolation de la région de la traversée à réaliser ;
c) détourage d'un ilot dans le matériau du substrat, par creusement dans le matériau du substrat d'au moins une tranchée débouchante s'étendant en direction transversale sur l'épaisseur du substrat de part en part de la région amincie,
   ladite tranchée débouchante s'étendant en direction latérale sur toute la périphérie de l'ilot à l'exception de ponts radiaux de matière maintenant l'ilot assujetti au reste du substrat ;
d) oxydation contrôlée du matériau du substrat, incluant une oxydation des parois latérales de la tranchée jusqu'à i) comblement complet du volume intérieur libre de la tranchée par croissance de l'oxyde dans ce volume intérieur libre, et ii) oxydation complète du matériau des ponts radiaux ; et
e) réalisation d'au moins une prise de contact pour ladite liaison électrique directement sur l'ilot.

Selon diverses variantes avantageuses de mise en œuvre de ce procédé :
- le procédé comprend successivement une étape de creusement, depuis une première face du substrat, d'une tranchée borgne s'étendant en direction transversale sur une fraction de l'épaisseur du substrat, suivie de ladite étape b) d'amincissement du substrat, depuis une seconde face opposée à la première face et en vis-à-vis de la tranchée borgne, sur une profondeur suffisante pour rejoindre la tranchée borgne et la rendre débouchante ;
- à l'étape b) l'amincissement est réalisé avec une largeur variable en fonction de la profondeur, élargie à proximité d'une première face du substrat et rétrécie vers une seconde face opposée du substrat, à proximité des ponts radiaux.

Dans une forme alternative de mise en œuvre du concept de l'invention, le procédé comprend, pour la réalisation du même type de traversée, les étapes successives suivantes :
a) obtention d'un substrat métallique dont l'épaisseur correspond à la dimension en direction transversale de la paroi comprenant la traversée à réaliser ;
b) détourage d'un ilot dans le matériau du substrat, depuis une première face du substrat, d'au moins une tranchée borgne s'étendant en direction transversale sur une fraction de l'épaisseur du substrat,
   ladite tranchée borgne s'étendant en direction latérale sur toute la périphérie de l'ilot ;
c) oxydation contrôlée du matériau du substrat, incluant une oxydation des parois latérales de la tranchée jusqu'à comblement de tout ou partie du volume intérieur libre de la tranchée par croissance de l'oxyde dans ce volume intérieur libre ;
d) creusement d'une gorge périphérique dans le substrat depuis une seconde face opposée à la première face,
   ladite gorge périphérique ayant en direction radiale une largeur et une position telles que la gorge une fois creusée rejoigne la tranchée borgne oxydée située en vis-à-vis, isolant ainsi électriquement l'ilot du reste du substrat ; et
e) réalisation d'au moins une prise de contact pour ladite liaison électrique directement sur l'ilot.

Avantageusement, à l'étape e) la position de la gorge périphérique creusée présente un décalage en direction transversale du contour de la gorge périphérique par rapport au contour de la tranchée borgne oxydée située en vis-à-vis, et il est prévu après creusement de la gorge une étape de gravure isotropique sélective du matériau non oxydé du substrat jusqu'à mise à nu du matériau oxydé de la tranchée, de manière à ainsi isoler électriquement l'ilot du reste du substrat.

Une variante du procédé ci-dessus comprend les étapes suivantes :
a) obtention d'un substrat métallique dont l'épaisseur correspond à la dimension en direction transversale de la paroi comprenant la traversée à réaliser ;
b) détourage dans le matériau du substrat d'un premier ilot, par creusement, depuis une première face du substrat, d'au moins une tranchée borgne s'étendant en direction transversale sur une fraction de l'épaisseur du substrat,
   ladite tranchée borgne s'étendant en direction latérale sur toute la périphérie du premier ilot ;
c) détourage dans le matériau du substrat d'un second ilot, par creusement, depuis une seconde face du substrat opposée à la première face, d'au moins une gorge borgne s'étendant en direction transversale sur une fraction de l'épaisseur du substrat,
   ladite gorge borgne s'étendant en direction latérale sur toute la périphérie du second ilot, le contour du second ilot étant proche du contour du premier ilot,
d) oxydation contrôlée du matériau du substrat, incluant une oxydation des parois de la tranchée et de la gorge sur une profondeur telle que, en direction latérale, la zone oxydée de la tranchée rejoigne la zone oxydée de la gorge, isolant ainsi électriquement les premier et second ilots du reste du substrat ; et
e) réalisation d'une prise de contact pour ladite liaison électrique directement sur l'un et/ou l'autre des premier et second ilots.

Dans une forme particulière de mise en œuvre de ce procédé, l'étape c) comprend le creusement, sur ladite première face, d'au moins un ensemble de deux tranchées borgnes concentriques encadrant en direction latérale chaque gorge borgne respective sur ladite seconde face opposée.

Selon des mises en œuvre avantageuses applicables aux diverses variantes des procédés exposés ci-dessus :
- le matériau du substrat métallique et de l'élément traversant métallique est un matériau biocompatible, biostable et résistant à la corrosion, notamment le titane ;
- le matériau électriquement isolant de la couche latérale périphérique est un oxyde du matériau du substrat métallique et de l'élément traversant ;
- ladite interface de couplage comprend au moins deux couches latérales périphériques en matériau électriquement isolant, concentriques ;
- dans ce dernier cas, la traversée peut comprendre une structure de condensateur couplée à ladite liaison électrique, ladite structure de condensateur comprenant trois couches latérales concentriques avec successivement : une couche conductrice intérieure formée dans le matériau du substrat, définissant une première armature du condensateur ; une couche intercalaire en matériau électriquement isolant, définissant un diélectrique du condensateur ; et une couche conductrice extérieure formée dans le matériau du substrat, définissant une seconde armature du condensateur.

On va maintenant décrire un exemple de mise en œuvre de la présente invention, en référence aux dessins annexés où les mêmes références désignent d'une figure à l'autre des éléments identiques ou fonctionnellement semblables.
La Figure 1 est une vue schématique d'une traversée avec les différents éléments qui la constituent.
La Figure 2 est une vue perspective de dessus d'un exemple de boitier de dispositif électronique muni d'une pluralité de traversées telles que celles de la Figure 1.
Les Figures 3 et 4 sont des vues en coupe transversale d'une traversée selon l'état de la technique, respectivement selon deux variantes différant par la façon de réaliser la prise de contact sur l'élément traversant.
La Figure 5 est une vue en coupe transversale de traversées réalisées par mise en œuvre de l'invention, selon deux exemples de réalisation possibles.
Les Figures 6 et 7 sont des vues, respectivement de dessus et en coupe transversale, illustrant un premier procédé selon l'invention de réalisation de traversées telles que celles illustrées Figure 5, au stade d'une étape initiale après gravure du substrat et avant oxydation de celui-ci.
La Figure 8 est une vue de dessus illustrant les phases successives (a) à (c) de l'étape d'oxydation du premier procédé selon l'invention, au niveau de l'un des ponts de matière visibles sur les Figures 6 et 7.
La Figure 9 est une vue en coupe transversale illustrant une variante de mise en œuvre du procédé de l'invention, après achèvement complet de l'étape d'oxydation.
La Figure 10 est une vue en coupe transversale illustrant les phases successives (a) à (c) de réalisation d'un contact électrique sur l'élément traversant, selon une première technique possible de prise de contact.
La Figure 11 est une vue en coupe transversale illustrant les phases successives (a) à (c) de réalisation d'un contact électrique sur l'élément traversant, selon une seconde technique possible de prise de contact.
La Figure 12 est une vue en coupe transversale illustrant les différentes phases (a) à (d) de réalisation d'une traversée par le premier procédé selon l'invention, selon deux variantes possibles pour l'amincissement du substrat dans la région de la traversée.
Les Figures 13 à 16 sont des vues en coupe transversale illustrant les différentes étapes successives d'un second procédé selon l'invention de réalisation d'une traversée.
Les Figures 17a et 17b sont des vues en coupe transversale illustrant, selon deux possibilités de mise en œuvre respectives, une variante du procédé des Figures 13 à 16, au stade du procédé où les tranchées borgnes viennent d'être gravées.
Les Figures 18a et 18b sont homologues des Figures 17a et 17b, après achèvement de l'étape d'oxydation contrôlée du matériau du substrat.

On va maintenant exposer, après avoir décrit l'état de la technique, divers exemples de traversées ainsi que deux procédés de réalisation de telles traversées à partir des enseignements de l'invention.

### Structure de traversée avec ilot central à maintien par pont de matière

Les Figures 1 à 4 illustrent un état de la technique.

Sur la Figure 1, on a illustré le principe d'une traversée, qui est destinée à permettre le passage d'une liaison électrique au travers d'une paroi électriquement conductrice comprenant un substrat métallique 10, typiquement en titane.

Le substrat métallique 10 comprend une face supérieure 12 et une face inférieure 14 (ces deux qualificatifs se référant uniquement à la présentation des figures, sans aucune connotation limitative ; il en sera de même pour les adjectifs "extérieur" et "intérieur" utilisés pour désigner respectivement ces deux mêmes côtés par rapport à un boitier dont le substrat 10 forme la paroi). La traversée 16 comprend un élément traversant métallique 18 électriquement conducteur, isolé entièrement du reste du substrat 10 par une région électriquement isolante 20, typiquement en céramique, entourant complètement l'élément traversant 18 sur sa périphérie et s'étendant de part en part du substrat 10, de la face 12 à la face 14. L'élément traversant 18 peut notamment être constitué d'un picot assurant une liaison électrique au travers du substrat 10. Dans d'autres réalisations, la surface apparente de l'élément traversant 18 est reliée à des pistes conductrices s'étendant sur la surface du substrat 10.

La Figure 2 illustre un exemple de dispositif dont la paroi extérieure du boitier est une paroi métallique, au travers de laquelle sont réalisées une pluralité de traversées 16.

Ces traversées 16 comprennent par exemple des picots 18 permettant d'assurer des liaisons électriques respectives, depuis l'extérieur du dispositif, à des bornes correspondantes de circuits internes du dispositif. On comprendra que le pas d'entraxe *p* entre picots adjacents est un élément important pour la conception du dispositif et que, dans un certain nombre de configurations, il peut être souhaitable de réduire autant que faire se peut cette valeur de pas d'entraxe *p.*

La Figure 3 illustre une structure de traversée telle que décrite dans le EP 2 377 573 A1 précité.

Une couche externe supérieure isolante 26 est formée en surface du substrat 10, au moins sur la face externe de celui-ci. Cette couche externe supérieure isolante 26 peut être notamment réalisée par oxydation du titane constituant le substrat 10 sur une profondeur contrôlée, ou par dépôt d'une couche de matériau isolant tel que du dioxyde de silicium en surface de l'épaisseur du substrat 10. L'épaisseur de la couche externe supérieure isolante est par exemple de l'ordre de 10 µm pour une épaisseur de boitier de l'ordre de 300 µm.

La structure de la traversée 16 comporte en outre un ilot conducteur constituant l'élément traversant 18. Cet ilot conducteur est ménagé dans l'épaisseur du substrat 10 par creusement d'une gorge 28 dans toute l'épaisseur du substrat 10, en laissant intacte la couche isolante 26 de manière que l'ilot 18 puisse être supporté par le pont de matière 30 formé par la couche isolante 26 entre la zone de l'ilot 18 et le reste du substrat 10. Dans le plan de la surface du boitier, la gorge 28 est creusée sur un contour fermé de manière à complètement isoler physiquement et donc électriquement l'ilot 18 du reste du substrat 10 sur toute sa périphérie. La couche externe isolante 26 constitue en outre une barrière hermétique entre les deux côtés du substrat, donc entre l'intérieur du boitier (dont ce substrat constitue la paroi) et l'environnement extérieur.

Pour assurer la prise de contact du côté extérieur, une ouverture 32 est formée dans la couche isolante externe 26, au droit de l'ilot 18, de manière à mettre à nu une zone sur laquelle pourra être par exemple brasé un fil 34 ou un picot, qui sera électriquement relié à l'ilot central 18 de la traversée et électriquement isolé du reste du substrat. Une liaison de type comparable peut être réalisée sur l'autre face du substrat, côté interne. Cette structure connue présente la particularité que l'ilot central 18 n'est relié, et mécaniquement supporté, que par le mince pont ou "diaphragme" de matière 30 de la couche d'oxyde supérieure isolante 26. Cette région, notamment le fond de la gorge 28, est particulièrement fragile et il peut apparaitre au cours du procédé de fabrication des défauts ou des micro-fissures susceptibles d'affaiblir ce pont 30, déjà fragile du fait de sa très faible épaisseur.

La Figure 4 illustre un perfectionnement proposé par le EP 2 873 437 A1 précité pour pallier cet inconvénient.

Ce document enseigne de déposer au-dessus de la couche externe isolante d'oxyde 26 une couche externe supplémentaire 36, isolante, par exemple par dépôt d'une métallisation de titane ou autre matériau tel que platine, palladium, or et leurs alliages, et ceci sur une épaisseur de l'ordre de quelques centaines de nanomètres à quelques micromètres. Cette structure procure un renforcement de la rigidité et de l'étanchéité du pont de matière 30 reliant mécaniquement l'ilot 18 au reste du substrat 10, du fait de l'augmentation de l'épaisseur totale de matière au droit de la gorge 28.

Cette structure renforcée présente cependant un certain nombre d'inconvénients qui demeurent, comme cela a été exposé en introduction : multiplication des étapes du procédé de réalisation avec corrélativement un coût de fabrication accru, nécessité de déposer des couches relativement épaisses (de l'ordre de 10 µm par couche) pour assurer la solidité mécanique avec risque de contrainte mécanique accrue et de déformation possible du substrat, etc.

On notera également que cette technique améliorée a pour conséquence d'augmenter de façon relativement importante la dimension *d* de l'emprise de la traversée 16 sur le substrat 10, ce qui implique un pas d'entraxe relativement important entre traversées adjacentes.

### Structure de traversée à isolation latérale verticale

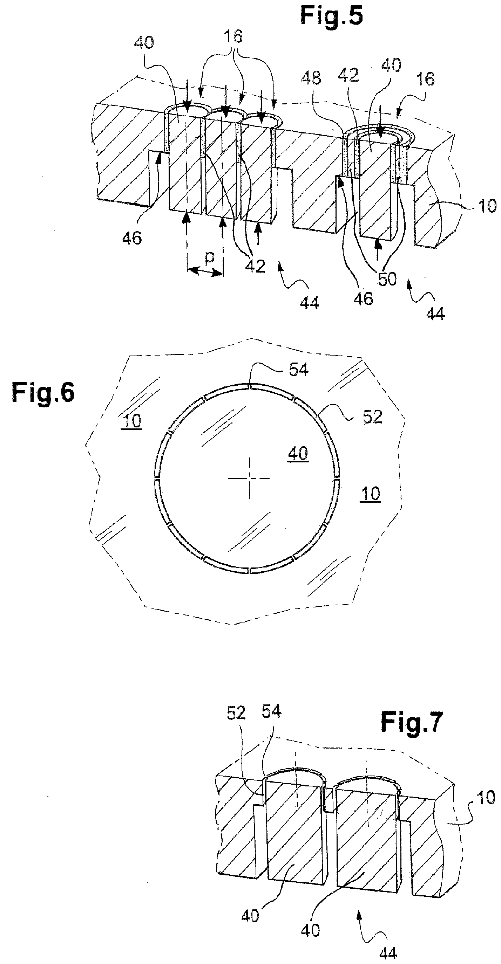

La Figure 5 est une vue en coupe transversale du substrat 10 montrant divers types de traversées à isolation latérale verticale, susceptibles d'être réalisées selon les enseignements de l'invention, avec :
- à gauche, trois traversées 16 attenantes, réalisées sur une région d'épaisseur réduite du substrat ;
- à droite, une traversée simple 16, également réalisée sur une région d'épaisseur réduite du substrat, avec une structure procurant une double isolation et/ou permettant l'intégration éventuelle d'un composant capacitif intégré de filtrage ou de découplage.

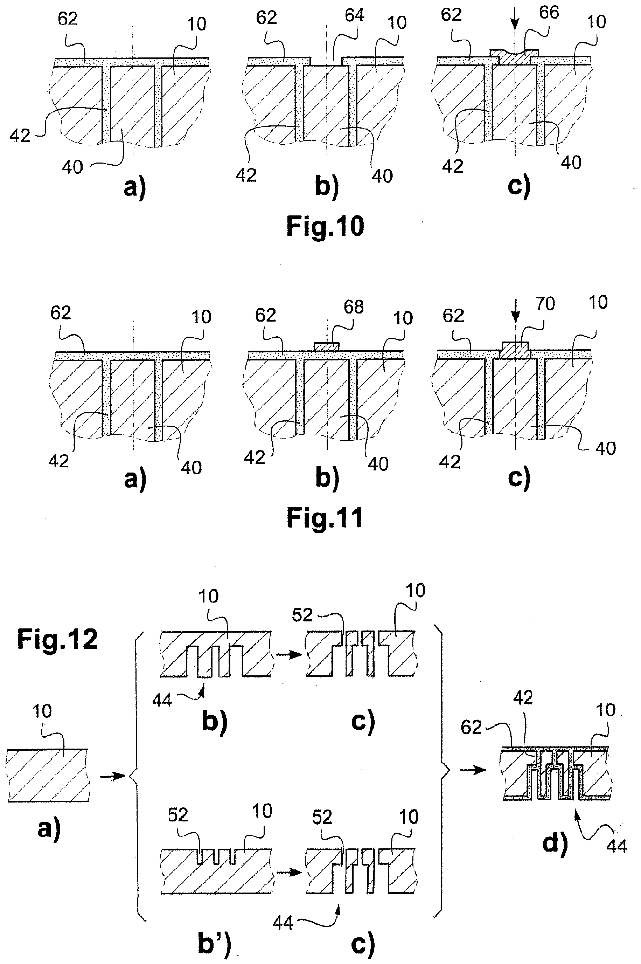

Sur les figures, les flèches verticales schématisent les emplacements où pourront être réalisées d'un côté et de l'autre du substrat les prises de contact sur l'élément traversant de chaque traversée 16, selon des techniques en elles-mêmes connues qui seront brièvement décrites par la suite, notamment en référence aux Figures 10 et 11.

Chaque traversée 16 comprend un élément traversant métallique 40, qui est un élément formé dans le matériau du substrat et s'étendant en direction transversale dans une région amincie, d'épaisseur réduite, du substrat (substrat dont l'épaisseur totale peut varier, typiquement mais de manière non limitative, entre 50 µm et 500 µm). Dans le sens latéral, l'élément traversant 40 est agencé comme un élément en forme d'ilot de contour fermé, physiquement et électriquement isolé du substrat.

On notera que dans la présente description le qualificatif "transversal" indique une direction correspondant à l'épaisseur du substrat, donc perpendiculaire à la surface de celui-ci, tandis qu'une direction "latérale" qualifiera une direction s'étendant suivant l'étendue du substrat, autrement dit une direction radiale par rapport à un axe transversal de la traversée.

En ce qui concerne le contour périphérique fermé de l'ilot définissant l'élément de traversée, ce contour peut être de toute forme : circulaire, polygonale (rectangulaire) ou quelconque, dès lors que par son caractère fermé elle isole complètement l'ilot du reste du substrat, physiquement et électriquement (le terme "périphérique" devant être entendu comme qualifiant un ilot structurellement séparé et électriquement isolé du reste du substrat).

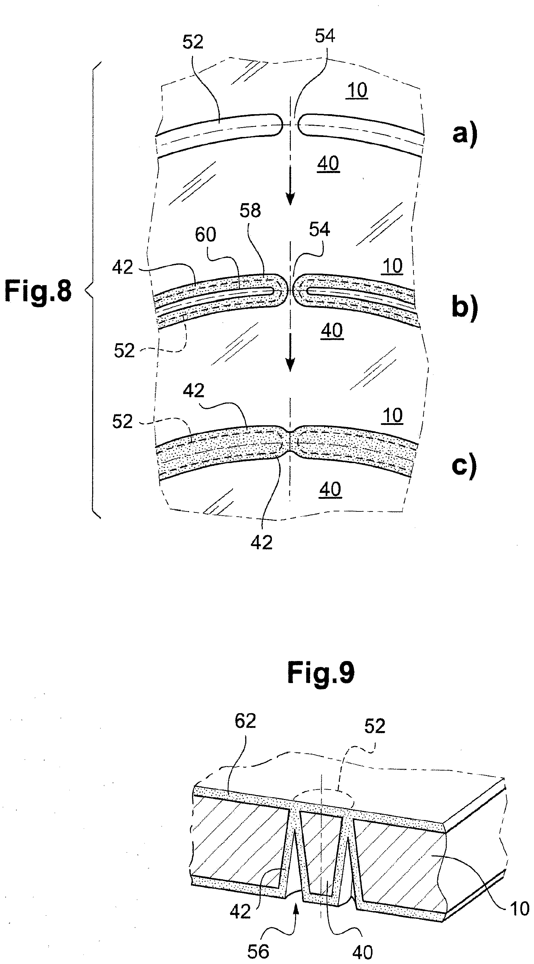

Par ailleurs, la génératrice définissant le contour de l'ilot ne s'étend pas nécessairement perpendiculairement au substrat : par exemple, le contour peut être cylindrique de révolution, mais aussi cylindrique quelconque, ou également conique (voir notamment Figure 9), pyramidal, ou de toute forme quelconque.

La traversée comprend en outre une interface de couplage de l'élément traversant 40 au reste du substrat 10, qui assure à la fois l'assujettissement mécanique de cet élément traversant au substrat et l'isolation électrique entre élément traversant et substrat.

Cette interface de couplage n'est pas assurée, comme dans l'état de la technique illustré Figures 3 et 4, par un pont ou diaphragme d'ancrage supérieur auquel l'élément traversant est suspendu dans un évidement du substrat : dans le cas présent, et de façon caractéristique, cette interface est obtenue par une couche latérale périphérique 42 en matériau électriquement isolant, enveloppant en direction latérale l'élément traversant 40 sur toute sa périphérie et s'étendant, en direction transversale, sur une partie amincie, d'épaisseur réduite, du substrat.

Les techniques de réalisation que l'on décrira plus loin permettent en particulier de réaliser un ensemble monolithique incorporant à la fois le substrat, l'élément traversant et la couche latérale. Dans cet ensemble, la couche latérale réalise, essentiellement et directement, une jonction (mécanique) directe et latérale de l'élément traversant 40 au substrat 10, d'où résulte à la fois i) l'assujettissement mécanique de l'élément traversant au substrat, et ii) l'isolation électrique entre l'élément traversant et le substrat.

Très avantageusement, le matériau de la couche latérale périphérique 42 est un oxyde du métal constituant le substrat 10, notamment de l'oxyde de titane TiO₂ : en effet, le titane et son oxyde sont des matériaux qui présentent les propriétés avantageuses de biocompatibilité, de biostabilité et de résistance à la corrosion qui les rendent particulièrement appropriés à un très grand nombre d'applications, notamment pour les dispositifs médicaux actifs en contact avec des tissus ou fluides corporels, tout particulièrement les dispositifs médicaux implantables.

L'oxydation contrôlée d'un métal tel que le titane est une technique en elle-même bien connue et maitrisée. Dans le cas de la présente invention, cette oxydation peut être réalisée à une température de l'ordre de 500 à 900 °C, sur une épaisseur typique de 0,1 à 15 µm.

Comme illustré sur la Figure 5, la(les) traversée(s) 16 est(sont) réalisée(s) sur une partie 44 d'épaisseur réduite du substrat, qui a été aminci depuis sa face inférieure (du côté intérieur du boitier) selon des techniques qui seront décrites par la suite notamment en référence à la Figure 12.

Avantageusement, comme on peut le remarquer au vu des figures, cet amincissement réalisé dans la région de la traversée est un amincissement partiel, de façon à laisser aux éléments traversants métalliques 40 leur épaisseur initiale, l'amincissement ne portant que sur les régions destinées à devenir des régions isolantes. Cette absence locale d'amincissement fait en sorte que les zones de contact/connexion restent au même niveau que le substrat, à la fois côté intérieur et côté extérieur de celui-ci.

Dans l'exemple illustré à gauche de la Figure 5, on a représenté une pluralité de traversées 16 semblables réalisées de la même manière, avec un pas d'entraxe *p* qui peut très réduit du fait que l'emprise en direction latérale est celle du seul élément traversant 40 avec sa couche latérale 42, sans aucun débordement périphérique. De plus, dans ce même exemple, les couches latérales périphériques 42 sont adjacentes les unes aux autres (c'est-à-dire qu'une couche isolante d'oxyde peut être commune à deux traversées adjacentes dans la région où celles-ci sont les plus proches), ce qui permet de réduire encore plus le pas d'entraxe *p* entre traversées adjacentes.

Une fois que l'amincissement de la partie d'épaisseur réduite 44 a été obtenu, un retrait ou épaulement 46 est présent entre la couche latérale périphérique 42 et le bord latéral de la partie d'épaisseur réduite 44. On notera également que lorsqu'une pluralité de traversées sont réalisées, celles-ci peuvent être, ou non, alignées, la compacité de la traversée selon l'invention permettant de réaliser des réseaux ou grilles de traversées selon des configurations très variées.

À droite de la Figure 5 est représenté un exemple de traversée simple comprenant deux couches latérales périphériques 42, 48 concentriques (le terme de "concentrique" étant entendu au sens le plus général, c'est-à-dire qu'une couche 48 extérieure entoure complètement une couche intérieure 42, les contours de ces couches n'étant pas nécessairement coaxiaux, ni même circulaires).

Le fait d'avoir plusieurs couches latérales périphériques permet de maximiser l'isolation électrique entre l'élément traversant 40 et le reste du substrat 10.

Cette configuration permet aussi d'éloigner physiquement l'élément traversant 40 et le reste du substrat 10, ce qui peut être intéressant dans certaines applications telles que les applications radiofréquence, où il est important de réduire le couplage électromagnétique entre structures voisines. En particulier (et aussi bien pour ce mode de réalisation que pour les autres) l'élément traversant 40 peut non seulement constituer une traversée, mais également une antenne émettrice/réceptrice radiofréquence isolée et éloignée du reste du substrat 10. Dans un tel cas, l'élément 40 pourra adopter toute forme de géométrie d'antenne connue, telle que boucle, zigzag, spirale, fourche, etc. On pourra se référer notamment au EP 2 873 437 A1 précité, qui décrit un tel type de composant RF intégré à un substrat et relié à une traversée monolithique réalisée au travers de ce substrat.

Cette configuration comprenant plusieurs couches latérales périphériques permet également, par un choix approprié des dimensions de ces couches, de réaliser une structure intégrée de condensateur de filtrage ou de découplage pouvant être associé à la traversée : en effet, l'alternance des couches concentriques métal/oxyde/métal correspond à une structure armature/diélectrique/armature d'un condensateur, la couche intercalaire faisant office de diélectrique. Tel est dans l'exemple illustré le cas des couches respectives 40/42/50 et 50/48/10.

Les paramètres de ce condensateur (capacité, tension de claquage) sont modulables par un choix approprié de l'épaisseur d'isolant et des dimensions de l'élément traversant (surface du contour périphérique et longueur en direction transversale). Ces paramètres peuvent être choisis en fonction du but recherché : soit pour réaliser un couplage contrôlé avec le substrat (par exemple à des fins de filtrage), soit au contraire pour découpler le plus possible les traversées d'avec le substrat. Dans ce dernier cas, il peut notamment être avantageux d'augmenter le nombre des interfaces concentriques (comme à droite de la Figure 5, où ces interfaces concentriques sont au nombre de deux) car dans ce cas les capacités (indésirables) se trouvent électriquement en série ce qui divise d'autant la capacité de couplage globale entre le substrat 10 et l'élément traversant principal 40, notamment lorsque celui-ci assure aussi une fonction d'antenne. Une autre possibilité consiste à réduire au maximum la fraction d'épaisseur des fines tranchées et de ne pas les combler. Le volume résiduel libre des rainures oxydées assure alors un plus grand découplage capacitif entre l'ilot traversant et le reste du substrat. Ces microcavités peuvent être laissées libres, ou bien comblées par le dépôt ultérieur d'un autre matériau de manière à rigidifier la structure. Avantageusement dans le cas où l'on souhaite une capacité électrique la plus faible possible, le matériau de remplissage ultérieur optionnel peut être choisi de manière à présenter une constante diélectrique plus faible que l'oxyde.

### Premier exemple de procédé selon l'invention de réalisation

### d'une structure de traversée à isolation latérale verticale

On va maintenant décrire divers procédés selon l'invention permettant d'obtenir des structures de traversée telles que celles décrites ci-dessus, avec les caractéristiques particulières suivantes :
- structure monolithique avec un ilot central conducteur provenant du substrat initial ;
- gravure sur chacune des faces du substrat, avec :
   - sur une face (la face inférieure sur les figures) un niveau de gravure permettant d'évider un accès large, d'ouverture typiquement comprise entre 25 et 300 µm en direction latérale, sur une profondeur importante, typiquement supérieure à 80 % de l'épaisseur initiale du substrat en direction transversale (substrat dont l'épaisseur totale est, typiquement limitativement, comprise entre 50 µm et 500 µm), et
   - sur l'autre face (la face supérieure sur les figures) un niveau de gravure permettant la réalisation de tranchées ultrafines, d'ouverture typiquement comprise entre 0,1 et 15 µm en direction latérale, sur une profondeur réduite, typiquement de 1 à 50 µm en direction transversale ;
- une isolation verticale latérale et circonférentielle de l'ilot par rapport à son substrat, cela sur une fraction de l'épaisseur de ce substrat (de préférence seulement dans une zone d'isolation de la région de la traversée à réaliser), l'isolation étant obtenue par une oxydation du substrat au niveau des tranchées ultrafines ; et
- métallisations supplémentaires éventuelles permettant une prise de contact électrique de meilleure qualité.

Comme on le verra, les traversées ainsi réalisées résolvent la triple problématique du maintien mécanique de l'ilot central, de son isolation électrique vis-à-vis du reste du substrat, et de l'étanchéité et de la robustesse mécanique finales du composant.

Un premier procédé caractéristique de la présente invention est illustré aux Figures 6 à 12.

Comme illustré aux Figures 6, 7 et 8(a), une fine tranchée débouchante 52 est gravée dans une région amincie 44, d'épaisseur réduite, du substrat. Plus précisément, le substrat 10 est creusé sur toute l'épaisseur de cette région amincie 44, le contour de la tranchée 52 définissant en direction latérale un ilot central correspondant à l'élément traversant 40 de la traversée à réaliser.

La fine tranchée débouchante 52 s'étendant sur toute l'épaisseur de la région amincie 44 de part en part de celle-ci, pour pouvoir maintenir en place l'élément central 40 il est prévu de laisser subsister de minces ponts radiaux 54 de matière non gravée. Le nombre et la configuration de ces ponts ou bras de matière non gravée peut être compris entre 1 et un nombre aussi élevé que souhaitable, ces ponts pouvant être symétriques ou non, et régulièrement répartis ou non. On notera incidemment que l'épaisseur réduite de la région 44 permet de réduire la longueur en direction transversale des ponts de matière 54.

Le creusement de la fine tranchée traversante 52 a ainsi permis de détourer l'ilot central correspondant à l'élément traversant 40, ce dernier étant maintenu en place par les ponts de matière 54. Cette situation correspond à celle de la Figure 8(a).

À ce stade, l'élément central est maintenu assujetti au reste du substrat par les ponts 54, mais il n'est pas isolé électriquement du substrat puisque les ponts laissent subsister un chemin conducteur. Il n'y a pas non plus d'étanchéité au travers de la structure entre l'élément central 40 et le reste du substrat 10, puisque les secteurs gravés de la fine tranchée 54 laissent subsister un volume intérieur libre, visible notamment sur la représentation agrandie de la Figure 8(a).

L'étape suivante, illustrée aux Figures 8(b), 8(c) et 9, consiste à réaliser simultanément l'isolation électrique et l'étanchéité de l'élément central 40 par une étape d'oxydation thermique contrôlée du substrat.

Cette étape va produire, concurremment :
- en profondeur, la croissance d'un front d'oxyde isolant consommant le métal du substrat (celui de l'élément central 40 comme celui du reste du substrat 10), comme illustré en 58 sur la Figure 8(b), où l'emplacement initial de la fine tranchée avant oxydation est représenté en tiretés en 52 ; et
- en surface, la croissance d'une épaisseur d'oxyde sur les parois de la fine tranchée 52, comme illustrée en 60 sur la Figure 8(b).

La poursuite de l'oxydation contrôlée va entrainer un comblement progressif du volume intérieur libre subsistant entre les deux parois en vis-à-vis de la tranchée 52, jusqu'à comblement complet de ce volume libre, comme illustré sur la Figure 8(c).

Par ailleurs, la dimension périphérique des bras de liaison 54 a été choisie suffisamment faible pour que, une fois la phase de comblement complet achevée, le métal du pont de matière 54 soit entièrement transformé en oxyde, comme illustré également sur la Figure 8(c). Les deux fronts en vis-à-vis de part et d'autre du pont de matière 54 se sont rejoints, isolant ainsi électriquement, et rendant étanche, l'élément central 40 vis-à-vis du reste du substrat 10.

La Figure 9 illustre le résultat obtenu dans une variante de mise en œuvre où, en coupe transversale, la tranchée 52 creusée dans la région 44 d'épaisseur réduite n'est pas une tranchée de largeur constante, autrement dit une tranchée avec des parois parallèles (comme dans le cas illustré Figure 7), mais une tranchée de largeur variable, avec des faces non parallèles s'écartant l'une de l'autre en allant vers l'une des faces du substrat. Ce non-parallélisme peut être notamment introduit par le procédé de fabrication qui génère un angle de pénétration. Le comblement de la tranchée ne se fait alors que sur une partie de l'épaisseur du substrat, choisie pour procurer le niveau requis à la fois d'assujettissement mécanique de l'élément central 40 au substrat 10 et d'isolation électrique de cet élément traversant 40 par rapport au substrat 10. Si la tenue mécanique n'est pas suffisante, la cavité subsistante 56 peut être remplie par dépôt d'un matériau tiers, isolant ou conducteur.

L'étape suivante consiste à réaliser une prise de connexion sur l'élément central 40.

En effet, celui-ci est isolé électriquement de tous côtés, notamment par la couche supérieure d'oxyde 62, et de même du côté opposé par la couche d'oxyde inférieure. Il est donc nécessaire, au niveau de l'élément traversant 40, de réaliser une continuité électrique entre les deux faces du substrat pour constituer la liaison électrique de la traversée.

La prise de connexion peut (dans ce mode de réalisation comme dans tous les autres) être réalisée sur l'une et l'autre des deux faces - supérieure et inférieure - de l'élément traversant central 40, avec report ou dépôt d'un élément de liaison conducteur (fil rapporté, piste en surface du substrat, etc.) destiné à assurer une liaison électrique à des éléments, circuits ou composants distants situés de part et d'autre du substrat, ceci de la même manière par exemple que les picots des traversées de la technique antérieure.

Mais la prise de connexion peut être aussi réalisée sur une face seulement de l'élément traversant, l'autre face de l'élément traversant étant une face active directement utilisable, par exemple pour constituer une électrode surfacique plaquée sur une face d'un boitier de dispositif, ou encore au niveau d'un capteur intégré à un dispositif. Cette configuration est particulièrement avantageuse pour la réalisation d'un dispositif implantable où cette électrode surfacique est destinée à venir en contact avec un tissu du patient porteur du dispositif.

Dans tous les cas, il convient de mettre à nu ou de laisser à nu chacune des faces supérieure et inférieure de l'élément traversant central 40.

Une première solution consiste, avant l'oxydation, à déposer un matériau inhibiteur d'oxydation tel que par exemple nitrure de titane, nitrure de silicium, tungstène, platine, niobium ou palladium ou toute combinaison de ces matériaux, sur une épaisseur de quelques dizaines à centaines de nanomètres, dans les régions de la surface du substrat où l'on ne souhaite pas voir croitre l'oxyde. Le contact électrique est alors directement obtenu après oxydation et élimination de la couche inhibitrice, avec éventuellement superposition ultérieure d'une couche supplémentaire d'un matériau métallique tel qu'or, platine, palladium, niobium, iridium ou toute combinaison de ces matériaux.

Une autre solution, illustrée Figure 10, consiste après achèvement de l'oxydation (Figure 10(a)) à éliminer localement la couche d'oxyde, par exemple par gravure plasma ou laser, jusqu'à mettre à nu la surface du matériau métallique de l'élément central 40, comme illustré en 64 sur la Figure 10(b).

La zone de contact électrique ainsi exposée peut, ici encore, être optimisée par dépôt, comme illustré en 66 sur la Figure 10(c), d'une couche métallique additionnelle telle qu'or, platine, palladium, niobium, iridium ou toute combinaison de ces matériaux. Cette couche métallique supplémentaire peut, en direction latérale, soit être confinée à la région 64 sans oxyde, soit déborder de celle-ci et recouvrir l'oxyde au-delà de la périphérie de la zone 64 (comme illustré en Figure 10(c)).

Une autre possibilité encore de réalisation de la prise de contact est illustrée Figure 11.

Après l'étape d'oxydation du substrat (Figure 11a), une couche d'un matériau approprié, tel qu'or, platine palladium, niobium, iridium ou toute combinaison de ces matériaux, est déposée au-dessus de la zone où l'on souhaite réaliser le contact, comme illustré en 68 sur la Figure 11(b). Un traitement thermique engendre alors une diffusion de ce matériau dans l'oxyde, comme illustré en 70 sur la Figure 11(c), ce qui a pour effet de rendre cet oxyde conducteur dans la zone sous-jacente.

Ces différentes techniques de réalisation de prise de contact sont en elles-mêmes connues et ne seront pas décrites plus en détail. Elles peuvent être mises en œuvre de la même façon de l'autre côté du substrat, de manière à définir une continuité électrique entre les deux faces, interne et externe, de l'élément traversant et réaliser ainsi la liaison électrique (ou chaque liaison électrique) de la traversée.

La Figure 12 illustre de façon schématique notamment la réalisation de la zone d'épaisseur réduite 44 du substrat, selon deux variantes possibles :
- dans une première variante, le substrat est d'abord aminci dans la région 44 pour lui donner une épaisseur réduite, puis la tranchée 52 est réalisée comme décrit précédemment, c'est-à-dire par creusement d'une fine tranchée traversante (Figures 12(a) à 12 (c) débouchant dans la face inférieure de la région amincie 44 ;
- dans une seconde variante, une fine tranchée borgne est creusée dans le substrat dans une fraction de son épaisseur, puis pour obtenir la région 44 d'épaisseur réduite, le substrat est aminci sur une profondeur suffisante pour rejoindre la fine tranchée borgne 52 qui avait été creusée depuis l'autre côté du substrat et rendre ainsi cette dernière traversante (Figures 12(a), 12(b') et 12(c')).

Ces étapes selon l'une ou l'autre variante sont ensuite suivies de l'étape d'oxydation du substrat (Figure 12(d)) permettant d'obtenir la structure voulue pour la réalisation de l'isolation des traversées sur la région d'épaisseur réduite 44 du substrat.

### Second exemple de procédé selon l'invention de réalisation d'une structure de traversée à isolation latérale verticale

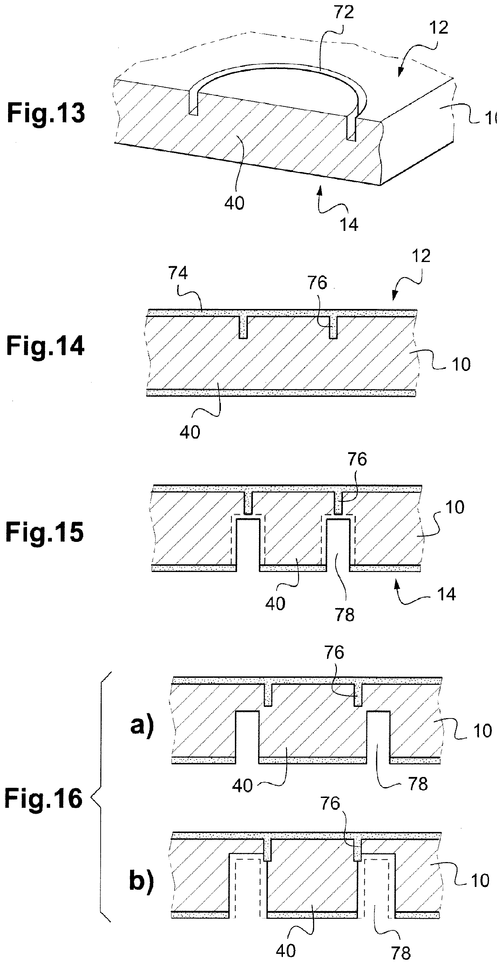

On va maintenant décrire en référence aux Figures 13 à 16 un second procédé selon l'invention permettant d'obtenir la structure de traversée monolithique dont les caractéristiques ont été exposées plus haut.

La première étape, illustrée Figure 13, consiste à détourer un ilot (correspondant à l'élément traversant de la traversée à réaliser) par creusement dans le matériau du substrat, depuis une première face de celui-ci, par exemple la face supérieure 12, d'une fine tranchée borgne 72 s'étendant en direction transversale sur une fraction de l'épaisseur du substrat 10. En direction latérale, cette fine tranchée s'étend sur toute la périphérie de l'ilot central 40 - c'est-à-dire que la fine tranchée 72 ne laisse pas subsister de ponts de matière, à la différence de la fine tranchée 52 illustrée Figure 6 du procédé précédent.

L'étape suivante, illustrée Figure 14, consiste à procéder à une oxydation contrôlée du matériau du substrat, incluant une oxydation des parois latérales de la fine tranchée jusqu'à comblement de tout ou partie du volume intérieur libre de celle-ci par croissance de l'oxyde.

À l'issue de cette étape d'oxydation, le substrat comporte sur l'une et l'autre de ses faces une couche de revêtement d'oxyde 74 (typiquement de quelques micromètres d'épaisseur) s'étendant, du côté de la face supérieure 12, le long de la fine tranchée 76 comblée totalement (comme illustré Figure 14) ou seulement partiellement. Dans le cas d'un remplissage partiel, de manière optionnelle le remplissage peut être rendu complet par le dépôt ultérieur d'une couche de remplissage isolante ou conductrice, de manière à notamment renforcer mécaniquement la structure.

L'étape suivante, illustrée Figure 15, consiste à creuser une gorge périphérique 78 depuis l'autre face du substrat, à savoir la face inférieure 14 dans l'exemple illustré. Cette gorge périphérique 78 peut être creusée sur une profondeur lui permettant d'atteindre la fine tranchée oxydée 76 et ainsi isoler complètement l'élément traversant central 40 ; sa largeur en direction transversale est généralement supérieure à celle de la fine tranchée borgne 72.

Pour éviter qu'une surgravure n'endommage l'oxyde isolant de la fine tranchée comblée 76, une variante avantageuse, illustrée Figure 15, consiste à arrêter le creusement de la gorge 78 légèrement avant d'atteindre la fine tranchée comblée 76 puis à achever la gravure par une attaque chimique plus sélective, illustrée en tiretés sur la Figure 15, cette attaque sélective attaquant essentiellement le substrat métallique 10 mais très peu l'oxyde de la fine tranchée comblée 76.

Toutefois, étant donné que les tolérances de profondeur de gravure entre le bord et le centre d'un même *wafer* portant un très grand nombre de composants distincts, certains endroits peuvent se trouver gravés plus profondément que d'autres.

Pour pallier cet inconvénient, et éviter une surgravure substantielle qui élargirait excessivement, notamment en direction latérale, les dimensions voulues, il est possible, comme illustré Figure 16, de décaler latéralement la gravure de la gorge 78 par rapport à la position de la fine tranchée comblée 76.

Ce décalage en direction latérale peut être réalisé soit vers l'intérieur soit, comme illustré Figure 16(a), vers l'extérieur, la profondeur de la gorge 78 gravée pouvant être inférieure, égale ou supérieure au niveau du fond de la fine tranchée comblée 76 située en vis-à-vis.

Dès lors, comme illustré en tiretés sur la Figure 16(b), une gravure isotropique du métal, chimique ou physique, se propageant dans toutes les directions permet dans toutes les configurations d'atteindre l'oxyde de la tranchée comblée 76 et d'isoler électriquement la partie centrale traversante 40 par rapport au reste du substrat. La profondeur de la gravure isotropique du métal est typiquement de l'ordre de 15 à 20 µm.

En effet, les tolérances d'alignement entre face avant et face arrière d'un même *wafer* sont très homogènes et très faibles (typiquement de 1 à 5 µm) quelle que soit la position du composant sur le *wafer,* ce qui réduit la profondeur de gravure isotrope nécessaire.

L'avantage de cette dernière variante est sa moindre sensibilité aux tolérances de profondeur de gravure, puisqu'il suffit d'atteindre une proximité minimale par rapport à l'oxyde de la fine tranchée comblée 76 pour compenser les variations dimensionnelles liées aux tolérances de fabrication.

De façon générale, et quelle que soit la variante de réalisation mise en œuvre, on notera que la couche isolante d'oxyde présente sur chaque face du substrat n'a plus aucune fonction ni électrique ni mécanique - à la différence des structures de l'art antérieur telles que celles illustrées Figures 3 et 4. Dès lors, cette couche d'oxyde peut être gravée totalement ou sélectivement pour mettre à nu le métal en surface du substrat. Le métal mis à nu peut être utilisé à diverses fins telles que contact électrique avec le substrat, soudure de la pièce réalisée sur un autre élément du dispositif, intégration d'une puce de circuit intégré, etc.

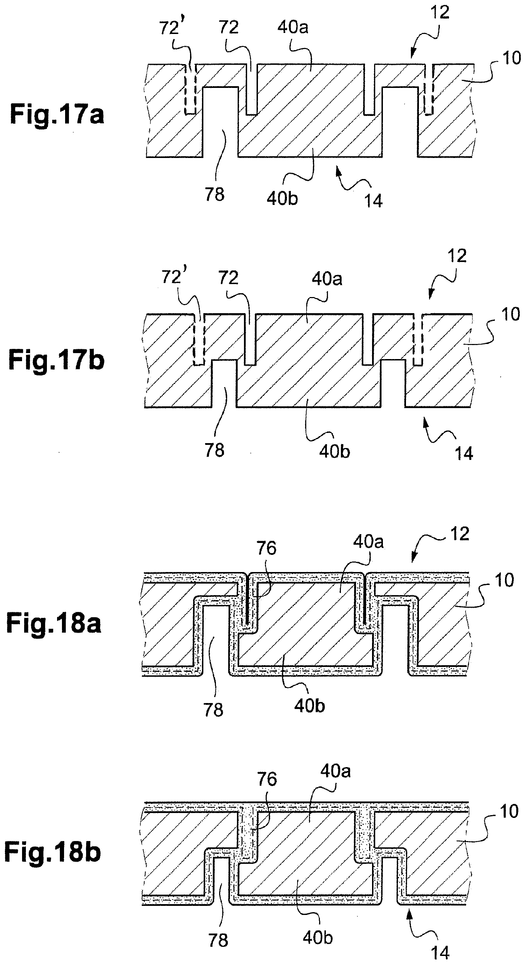

Les Figures 17a, 17b, 18a et 18b sont des vues en coupe transversale illustrant, selon deux possibilités différentes de mise en œuvre, une variante du procédé des Figures 13 à 16, respectivement au stade du procédé où la fine tranchée borgne 72 et la gorge 78 viennent d'être gravées (Figures 17a et 17b) et après achèvement de l'étape d'oxydation contrôlée du matériau du substrat

Dans cette variante, où la fine tranchée 72 gravée sur une face est décalée par rapport à la gorge 78 gravée sur l'autre face, l'isolation électrique entre les gravures (et donc entre l'ilot central et le reste du substrat) est établie par une oxydation 76 de l'interstice entre la tranchée 72 et la gorge 78. Ceci peut être réalisé par gravure de la tranchée borgne 72 et de la gorge 78 soit en décalage avec chevauchement vertical (Figure 17a), soit en décalage en proximité verticale (Figure 17b).

L'ordre de gravure de la tranchée borgne 72 et de la gorge 78 sur une face puis l'autre est indifférent. La tranchée 72 et la gorge 78 peuvent être décalées dans des sens différents (décalage interne, décalage externe, décalage à cheval). Chacune de la tranchée borgne 72 et de la gorge 78 définit un ilot respectif 40a, 40b et ces deux ilots, lorsqu'ils seront électriquement isolés ensemble du reste du substrat, formeront l'élément conducteur central 40 de la traversée.

La profondeur de gravure de la tranchée borgne 72 et de la gorge 78 doit être suffisante de part et d'autre pour définir une séparation très fine entre les deux (dont la dimension peut varier, typiquement mais de manière non limitative, entre 0,5 µm et 25 µm).

Le décalage définissant l'interstice entre la tranchée borgne 72 et la gorge 78 sera suffisamment fin pour que cet interstice soit intégralement oxydé (comme en 76 sur les figures), devenant ainsi électriquement isolant.

Dans la mise en œuvre illustrée Figure 18b, l'une au moins de la tranchée borgne 72 et de la gorge 78, de préférence la tranchée 72 comme illustré

Figures 18a et 18b, peut être suffisamment étroite pour que son volume soit intégralement rempli par l'oxyde pendant l'oxydation thermique (comme en 76 Figure 18b). SI ce n'est pas le cas (comme en Figure 18a), le volume résiduel libre de la (des) tranchée(s) oxydée(s) peut être comblé -ou non - par le dépôt ultérieur d'un autre matériau, de manière à rigidifier la structure.

Enfin, pour pallier tout défaut d'alignement de la gravure d'une face vis-à-vis de l'autre qui génèrerait d'un côté une toile trop épaisse pour une oxydation complète de celle-ci, et de l'autre une toile trop fine ou inexistante, il peut être prévu un moyen de compensation d'alignement par réalisation sur une face d'un jeu de deux tranchées fines concentriques 72, 72' (comme cela est illustré sur les Figures 17a et 17b) encadrant chaque gorge sur la face opposée. De la sorte, il y aura toujours de part et d'autre de l'ilot central au moins une toile ayant les dimensions voulues, quelles que soient les tolérances d'alignement entre les gravures respectives de chaque face.

## Revendications

1. Un procédé de réalisation d'une traversée (16) hermétique et électriquement isolante pour le passage d'une liaison électrique au travers d'une paroi métallique d'un dispositif électrique, **caractérisé en ce qu'**il comprend les étapes suivantes :
a) obtention d'un substrat métallique (10) dont l'épaisseur correspond à la dimension en direction transversale de la paroi comprenant la traversée à réaliser ;
b) amincissement du substrat, au moins dans une zone d'isolation de la région de la traversée à réaliser ;
c) détourage d'un ilot (40) dans le matériau du substrat, par creusement dans le matériau du substrat d'au moins une tranchée débouchante (52) s'étendant en direction transversale sur l'épaisseur du substrat de part en part de la région amincie (44),
ladite tranchée débouchante s'étendant en direction latérale sur toute la périphérie de l'ilot à l'exception de ponts radiaux (54) de matière maintenant l'ilot assujetti au reste du substrat ;
d) oxydation contrôlée du matériau du substrat, incluant une oxydation (58, 60) des parois latérales de la tranchée jusqu'à i) comblement complet du volume intérieur libre de la tranchée par croissance de l'oxyde dans ce volume intérieur libre, et ii) oxydation complète du matériau des ponts radiaux ; et
e) réalisation d'au moins une prise de contact pour ladite liaison électrique directement sur l'ilot.

2. Le procédé de la revendication 1, comprenant successivement :
- une étape de creusement, depuis une première face du substrat, d'une tranchée borgne s'étendant en direction transversale sur une fraction de l'épaisseur du substrat, suivie de
- ladite étape b) d'amincissement du substrat, depuis une seconde face opposée à la première face et en vis-à-vis de la tranchée borgne, sur une profondeur suffisante pour rejoindre la tranchée borgne et la rendre débouchante.

3. Le procédé de la revendication 1, dans lequel à l'étape b) l'amincissement est réalisé avec une largeur variable en fonction de la profondeur, élargie à proximité d'une première face du substrat et rétrécie vers une seconde face opposée du substrat, à proximité des ponts radiaux.

4. Un procédé de réalisation d'une traversée (16) hermétique et électriquement isolante pour le passage d'une liaison électrique au travers d'une paroi métallique d'un dispositif électrique, **caractérisé en ce qu'**il comprend les étapes successives suivantes :
a) obtention d'un substrat métallique (10) dont l'épaisseur correspond à la dimension en direction transversale de la paroi comprenant la traversée à réaliser ;
b) détourage d'un ilot (40) dans le matériau du substrat, par creusement, depuis une première face (12) du substrat, d'au moins une tranchée borgne (72) s'étendant en direction transversale sur une fraction de l'épaisseur du substrat,
ladite tranchée borgne s'étendant en direction latérale sur toute la périphérie de l'ilot ;
c) oxydation contrôlée du matériau du substrat, incluant une oxydation des parois latérales de la tranchée jusqu'à comblement de tout ou partie du volume intérieur libre de la tranchée par croissance de l'oxyde dans ce volume intérieur libre ;
d) creusement d'une gorge périphérique (78) dans le substrat depuis une seconde face (14) opposée à la première face,
ladite gorge périphérique ayant en direction radiale une largeur et une position telles que la gorge (78) une fois creusée rejoigne la tranchée borgne oxydée (76) située en vis-à-vis, isolant ainsi électriquement l'ilot du reste du substrat ; et
e) réalisation d'au moins une prise de contact pour ladite liaison électrique directement sur l'ilot.

5. Le procédé de la revendication 4, dans lequel :
- à l'étape e) la position de la gorge périphérique (78) creusée présente un décalage en direction transversale du contour de la gorge périphérique par rapport au contour de la tranchée borgne oxydée (76) située en vis-à-vis ; et
- il est prévu après creusement de la gorge une étape de gravure isotropique sélective du matériau non oxydé du substrat jusqu'à mise à nu du matériau oxydé de la tranchée, de manière à ainsi isoler électriquement l'ilot du reste du substrat.

6. Un procédé de réalisation d'une traversée (16) hermétique et électriquement isolante pour le passage d'une liaison électrique au travers d'une paroi métallique d'un dispositif électrique, **caractérisé en ce qu'**il comprend les étapes suivantes :
a) obtention d'un substrat métallique (10) dont l'épaisseur correspond à la dimension en direction transversale de la paroi comprenant la traversée à réaliser ;
b) détourage dans le matériau du substrat d'un premier ilot (40a), par creusement, depuis une première face (12) du substrat, d'au moins une tranchée borgne (72) s'étendant en direction transversale sur une fraction de l'épaisseur du substrat,
ladite tranchée borgne (72) s'étendant en direction latérale sur toute la périphérie du premier ilot (40a) ;
c) détourage dans le matériau du substrat d'un second ilot (40b), par creusement, depuis une seconde face (14) du substrat opposée à la première face, d'au moins une gorge borgne (78) s'étendant en direction transversale sur une fraction de l'épaisseur du substrat,
ladite gorge borgne (78) s'étendant en direction latérale sur toute la périphérie du second ilot (40b), le contour du second ilot (40b) étant proche du contour du premier ilot (40a),
d) oxydation contrôlée du matériau du substrat, incluant une oxydation des parois de la tranchée (72) et de la gorge (78) sur une profondeur telle que, en direction latérale, la zone oxydée de la tranchée rejoigne la zone oxydée de la gorge, isolant ainsi électriquement les premier et second ilots (40a, 40b) du reste du substrat ; et
e) réalisation d'une prise de contact pour ladite liaison électrique directement sur l'un et/ou l'autre des premier et second ilots (40a, 40b).

7. Le procédé de la revendication 6, dans lequel l'étape c) comprend le creusement, sur ladite première face (12), d'au moins un ensemble de deux tranchées borgnes concentriques (72, 72') encadrant en direction latérale chaque gorge borgne (78) respective sur ladite seconde face (14) opposée.

8. Le procédé de l'une des revendications 1, 4 ou 6, dans lequel le matériau du substrat métallique (10) et de l'élément traversant métallique (40) est un matériau biocompatible, biostable et résistant à la corrosion.

9. Le procédé de la revendication 8, dans lequel le matériau biocompatible, biostable et résistant à la corrosion est le titane.

10. Le procédé de l'une des revendications 1, 4 ou 6, dans lequel le matériau électriquement isolant de la couche latérale périphérique (42) est un oxyde du matériau du substrat métallique (10) et de l'élément traversant (40).

11. Le procédé de l'une des revendications 1, 4 ou 6, dans lequel ladite interface de couplage comprend au moins deux couches latérales périphériques (42, 48) en matériau électriquement isolant, concentriques.

12. Le procédé de la revendication 11, dans lequel la traversée comprend une structure de condensateur couplée à ladite liaison électrique, ladite structure de condensateur comprenant trois couches latérales (40, 42, 10 ; 40, 42, 50 ; 50, 48, 10) concentriques avec successivement :
- une couche conductrice intérieure (40 ; 40 ; 50) formée dans le matériau du substrat, définissant une première armature du condensateur ;
- une couche intercalaire (42 ; 42 ; 50) en matériau électriquement isolant, définissant un diélectrique du condensateur ; et
- une couche conductrice extérieure (10 ; 50 ; 10) formée dans le matériau du substrat, définissant une seconde armature du condensateur.

## Patentansprüche

1. Verfahren zur Herstellung einer hermetischen und elektrisch isolierten Durchführung (16) für das Durchführen einer elektrischen Leitung durch eine metallische Wand einer elektrischen Vorrichtung hindurch, **dadurch gekennzeichnet, dass** es die folgenden Schritte umfasst:
a) Erhalten eines metallischen Substrats (10), dessen Dicke der Abmessung der Wand in Querrichtung entspricht, welche die herzustellende Durchführung umfasst,
b) Verdünnen des Substrats, wenigstens in einer Zone zum Isolieren des Bereichs der herzustellenden Durchführung;
c) Ausschneiden einer Insel (40) aus dem Material des Substrats, durch Austiefen in dem Material des Substrats wenigstens eines ausmündenden Grabens (52), der sich in Querrichtung über die Dicke des Substrats von einer Seite zur anderen des verdünnten Bereichs (44) erstreckt, wobei der ausmündende Grabens sich in seitlicher Richtung über den gesamten Umfang der Insel mit Ausnahme von radialen Materialbrücken (54) erstreckt, welche die Insel an dem Rest des Substrats befestigt halten;
d) kontrolliertes Oxidieren des Materials des Substrats, umfassend eine Oxidation (58, 60) der Seitenwände des Grabens bis zum (i) vollständigen Verfüllen des freien Innenraums des Grabens durch Anwachsen des Oxids in diesem freien Innenraum und ii) vollständigen Oxidieren des Materials der radialen Brücken; und
e) Herstellen wenigstens eines Steckkontakts für die elektrische Leitung direkt auf der Insel.

2. Verfahren nach Anspruch 1, nacheinander umfassend:
- einen Schritt des Austiefens, von einer ersten Fläche des Substrats aus, eines Blindgrabens, der sich in Querrichtung über einen Bruchteil der Dicke des Substrats erstreckt, gefolgt von
- dem Schritt b) des Verdünnens des Substrats, von einer zweiten Fläche aus, die der ersten Fläche gegenüber gelegen ist, und dem Blindgraben gegenüber, über eine Tiefe, die ausreicht, um eine Verbindung zum Blindgraben herzustellen und ihn ausmünden zu lassen.

3. Verfahren nach Anspruch 1, bei dem in Schritt b) das Verdünnen mit einer von der Tiefe abhängigen variablen Breite durchgeführt wird, die in der Nähe einer ersten Fläche des Substrat verbreitert ist und zu einer zweiten gegenüberliegenden Fläche des Substrats, in der Nähe der radialen Brücken, hin verengt ist.

4. Verfahren zur Herstellung einer hermetischen und elektrisch isolierten Durchführung (16) für das Durchführen einer elektrischen Leitung durch eine metallische Wand einer elektrischen Vorrichtung hindurch, **dadurch gekennzeichnet, dass** es die folgenden aufeinanderfolgenden Schritte umfasst:
a) Erhalten eines metallischen Substrats (10), dessen Dicke der Abmessung der Wand in Querrichtung entspricht, welche die herzustellende Durchführung umfasst,
b) Ausschneiden einer Insel (40) aus dem Material des Substrats, durch Austiefen, von einer ersten Fläche (12) des Substrats aus, wenigstens eines Blindgrabens (72), der sich in Querrichtung über einen Bruchteil der Dicke des Substrats erstreckt, wobei der Blindgraben sich in seitlicher Richtung über den gesamten Umfang der Insel erstreckt;
c) kontrolliertes Oxidieren des Materials des Substrats, einschließlich einer Oxidation der Seitenwände des Grabens bis zum vollständigen oder teilweisen Verfüllen des freien Innenraums des Grabens durch Anwachsen des Oxids in diesem freien Innenraum;
d) Austiefen einer umlaufenden Nut (78) in dem Substrat von einer zweiten Fläche (14) aus, die der ersten Fläche gegenüberliegt, wobei die umlaufende Nut in radialer Richtung eine derartige Breite und eine derartige Position aufweist, dass die Nut (78), sobald sie ausgetieft ist, die Verbindung mit dem gegenüber gelegenen oxidierten Blindgraben (76) herstellt und so die Insel von dem Rest des Substrats elektrisch isoliert; und
e) Herstellen wenigstens eines Steckkontakts für die elektrische Leitung direkt auf der Insel.

5. Verfahren nach Anspruch 4, bei dem:
- in Schritt a) die Position der ausgetieften umlaufenden Nut (78) einen Versatz in Querrichtung der Kontur der umlaufenden Nut gegenüber der Kontur des gegenüber gelegenen oxidierten Blindgrabens (76) aufweist; und
- nach Austiefen der Nut ein Schritt des selektiven isotropischen Ätzens des nicht oxidierten Materials des Substrats vorgesehen ist, bis das oxidierte Material des Grabens freigelegt ist, wodurch die Insel von dem Rest des Substrats elektrisch isoliert wird.

6. Verfahren zur Herstellung einer hermetischen und elektrisch isolierten Durchführung (16) für das Durchführen einer elektrischen Leitung durch eine metallische Wand einer elektrischen Vorrichtung hindurch, **dadurch gekennzeichnet, dass** es die folgenden Schritte umfasst:
a) Erhalten eines metallischen Substrats (10), dessen Dicke der Abmessung der Wand in Querrichtung entspricht, welche die herzustellende Durchführung umfasst,
b) Ausschneiden aus dem Material des Substrats einer ersten Insel (40a), durch Austiefen, von einer ersten Fläche (12) des Substrats aus, wenigstens eines Blindgrabens (72), der sich in Querrichtung über einen Bruchteil der Dicke des Substrats erstreckt, wobei der Blindgraben (72) sich in seitlicher Richtung über den gesamten Umfang der ersten Insel (40a) erstreckt;
c) Ausschneiden aus dem Material des Substrats einer zweiten Insel (40b), durch Austiefen, von einer zweiten Fläche (14) des Substrats aus, die der ersten Fläche gegenüberliegt, wenigstens eines Blindgrabens (78), der sich in Querrichtung über einen Bruchteil der Dicke des Substrats erstreckt, wobei der Blindgraben (78) sich in seitlicher Richtung über den gesamten Umfang der zweiten Insel (40b) erstreckt, wobei die Kontur der zweiten Insel (40b) der Kontur der ersten Insel (40a) nahe kommt,
d) kontrolliertes Oxidieren des Materials des Substrats, umfassend eine Oxidation der Wände des Grabens (72) und der Nut (78) über eine derartige Tiefe, dass, in seitlicher Richtung, die oxidierte Zone des Grabens die Verbindung mit der oxidierten Zone der Nut herstellt und so die erste und zweite Insel (40a, 40b) von dem Rest des Substrats elektrisch isoliert; und
e) Herstellen eines Steckkontakts für die elektrische Leitung direkt auf der einen und/oder der anderen der ersten und zweiten Insel (40a, 40b).

7. Verfahren nach Anspruch 6, bei dem der Schritt c) das Austiefen, auf der ersten Fläche (12), wenigstens einer Anordnung aus zwei konzentrischen Blindgräben (72, 72') umfasst, die in seitlicher Richtung jede jeweilige Blindnut (78) auf der zweiten gegenüberliegenden Fläche (14) einrahmen.

8. Verfahren nach einem der Ansprüche 1, 4 oder 6, wobei das Material des metallischen Substrats (10) und des metallischen Durchführungselements (40) ein biokompatibles, biostabiles und korrosionsbeständiges Material ist.

9. Verfahren nach Anspruch 8, bei dem das biokompatible, biostabile und korrosionsbeständige Material Titan ist.

10. Verfahren nach einem der Ansprüche 1, 4 oder 6, wobei das elektrisch isolierende Material der umlaufenden seitlichen Schicht (42) ein Oxid des Materials des metallischen Substrats (10) und des metallischen Durchführungselements (40) ist.

11. Verfahren nach einem der Ansprüche 1, 4 oder 6, wobei die Verbindungsschnittstelle wenigstens zwei konzentrische umlaufende seitliche Schichten (42, 48) aus elektrisch isolierendem Material umfasst.

12. Verfahren nach Anspruch 11, bei dem die Durchführung eine Kondensatorstruktur umfasst, die mit der elektrischen Leitung verbunden ist, wobei die Kondensatorstruktur drei konzentrische seitliche Schichten (40, 42, 10; 40, 42, 50; 50, 48, 10) umfasst, mit aufeinander folgend:
- einer inneren leitenden Schicht (40; 40; 50), die in dem Material des Substrats gebildet ist und eine erste Belegung des Kondensators bildet;
- einer Zwischenschicht (42; 42; 50) aus elektrisch isolierendem Material, die einen Isolator des Kondensators bildet; und
- einer äußeren leitenden Schicht (10; 50; 10), die in dem Material des Substrats gebildet ist und eine zweite Belegung des Kondensators bildet.

## Claims

1. A method of making a hermetic and electrically isolating feedthrough (16) for the passage of an electrical connection through a metal wall of an electrical device, **characterized by** comprising the following steps:
a) obtaining a metallic substrate (10) whose thickness corresponds to the transverse size of the wall comprising the feedthrough to be made;
b) thinning down the substrate, at least in a zone of isolation of the area of the feedthrough to be made;
c) shaping an islet (40) into the substrate material, by hollowing out from the substrate material a through-trench (52) extending transversally through the substrate thickness, right through the thinned region (44), said through-trench extending transversally over the whole periphery of the islet with the exception of radial bridges (54) of material holding the islet mechanically secured to the remainder of the substrate;
d) performing a controlled oxidation of the substrate material, including an oxidation (58, 60) of the lateral walls of the trench up to i) complete filling of the free inner volume of the trench by growth of the oxide in this free inner volume, and ii) complete oxidation of the material of the radial bridges; and
e) making at least one contact arrangement for said electrical connection directly on the islet.

2. The method of claim 1, comprising successively:
- a step of hollowing out, from a first face of the substrate, a blind trench extending transversally through a fraction of the substrate thickness, followed by
- said step b) of thinning down the substrate, from a second face opposite to the first face and facing the blind trench, over a sufficient depth to reach the blind trench and make the latter through-going.

3. The method of claim 1, wherein, at step b), the thinning down is made with a width varying as a function of the depth, increased near a first face of the substrate and reduced toward a second, opposite face of the substrate, near the radial bridges.

4. A method of making a hermetic and electrically isolating feedthrough (16) for the passage of an electrical connection through a metal wall of an electrical device, **characterized by** comprising the following successive steps:
a) obtaining a metallic substrate (10) whose thickness corresponds to the transverse size of the wall comprising the feedthrough to be made;
b) shaping an islet (40) into the substrate material, by hollowing out, from a first face (12) of the substrate, at least one blind trench (72) extending transversally through a fraction of the substrate thickness, said blind trench extending laterally over the whole periphery of the islet;
c) performing a controlled oxidation of the substrate material, including an oxidation of the lateral walls of the trench up to filling all or part of the free inner volume of the trench by growth of the oxide in this free inner volume;
d) hollowing out a peripheral groove (78) from the substrate, from a second face (14) opposite to the first face,
said peripheral groove having radially such width and position that the groove (78), once hollowed out, reaches the oxidized blind trench (76) facing it, hence electrically isolating the islet from the remainder of the substrate; and
e) making at least one contact arrangement for said electrical connection directly on the islet.

5. The method of claim 4, wherein:
- at step e), the position of the hollowed-out peripheral groove (78) shows a transverse offset of the peripheral groove contour with respect to the contour of the oxidized blind trench (76) facing it, and
- it is provided, after the groove has been hollowed out, a step of selective isotropic engraving of the non-oxidized material of the substrate up to exposing the oxidized material of the trench, so as to hence electrically isolate the islet from the remainder of the substrate.

6. A method of making a hermetic and electrically isolating feedthrough (16) for the passage of an electrical connection through a metal wall of an electrical device, **characterized by** comprising the following successive steps:
a) obtaining a metallic substrate (10) whose thickness corresponds to the transverse size of the wall comprising the feedthrough to be made;
b) shaping a first islet (40a) into the substrate material, by hollowing out, from a first face (12) of the substrate, at least one blind trench (72) extending transversally through a fraction of the substrate thickness, said blind trench (72) extending laterally over the whole periphery of the first islet (40a);
c) shaping a second islet (40b) into the substrate material, by hollowing out, from a second face (14) of the substrate, opposite to the first face, at least one blind groove (78) extending transversally through a fraction of the substrate thickness,
said blind groove (78) extending laterally over the whole periphery of the second islet (40b), the contour of the second islet (40b) being close to the contour of the first islet (40a);
d) performing a controlled oxidation of the substrate material, including an oxidation of the walls of the trench (72) and of the groove (78) through such a depth that, laterally, the oxidized area of the trench reaches the oxidized area of the groove, hence electrically isolating the first and second islets (40a, 40b) from the remainder of the substrate; and
e) making a contact arrangement for said electrical connection directly on one and/or the other of the first and second islets (40a, 40b).

7. The method of claim 6, wherein step c) comprises hollowing out, from said first face (12), at least a set of two concentric blind trenches (72, 72') laterally surrounding each respective blind groove (78) on said second, opposite face (14).

8. The method of any of claims 1, 4 or 6, wherein the material of the metallic substrate (10) and of the metal through-element (40) is a material that is biocompatible, biostable and resistant to corrosion.

9. The method of claim 8, wherein the biocompatible, biostable and corrosion-resistant material is titanium.

10. The method of any of claims 1, 4 or 6, wherein the electrically isolating material of the peripheral lateral layer (42) is an oxide of the material of the metallic substrate (10) and of the through-element (40).

11. The method of any of claims 1, 4 or 6, wherein said coupling interface comprises at least two concentric peripheral lateral layers (42, 48) made of an electrically isolating material.

12. The method of claim 11, wherein the feedthrough comprises a capacitor structure coupled to said electrical connection, said capacitor structure comprising three concentric lateral layers (40, 42, 10 ; 40, 42, 50 ; 50, 48, 10) with, successively:
- a lower conductive layer (40 ; 40 ; 50) formed in the substrate material, defining a first electrode for the capacitor;
- an intermediate layer (42 ; 42 ; 50) made of an electrically isolating material, defining a dielectric of the capacitor; and
- an external conductive layer (10 ; 50 ; 10) formed in the substrate material, defining a second electrode for the capacitor.
